# EUROPEAN PATENT SPECIFICATION

(11) **EP 3 517 041 B1**
(45) Date of publication and mention of the grant of the patent: **11.08.2021**
(21) Application number: 19163393.2
(22) Date of filing: 25.06.2015
(51) Int. Cl.: A61B 6/03, A61B 6/14, A61B 6/00

(54) **X-RAY IMAGING UNIT FOR A MEDICAL IMAGING**
RÖNTGENBILDGEBUNGSEINHEIT ZUR MEDIZINISCHEN BILDGEBUNG
UNITÉ D'IMAGERIE À RAYONS X POUR UNE IMAGERIE MÉDICALE

(30) Priority: 26.06.2014 FI 20145617
(43) Date of publication of application: 31.07.2019
(62) Divisional of application: 15173733.5
(73) Proprietor: PaloDEx Group Oy, 04301 Tuusula (FI)
(72) Inventor: SUURONEN, Esa, 04200 Kerava (FI); VARTIAINEN, Sami, 01620 Vantaa (FI); ANTIKAINEN, Ari, 04150 Martinkylä (FI)
(74) Representative: Berggren Oy

(56) References cited:
- EP-A1- 2 210 559
- EP-A1- 2 609 861
- US-A1- 2011 142 199
- US-B1- 6 466 641

## Description

### Technical field

The application relates generally to an X-ray imaging unit for a medical imaging.

### Background

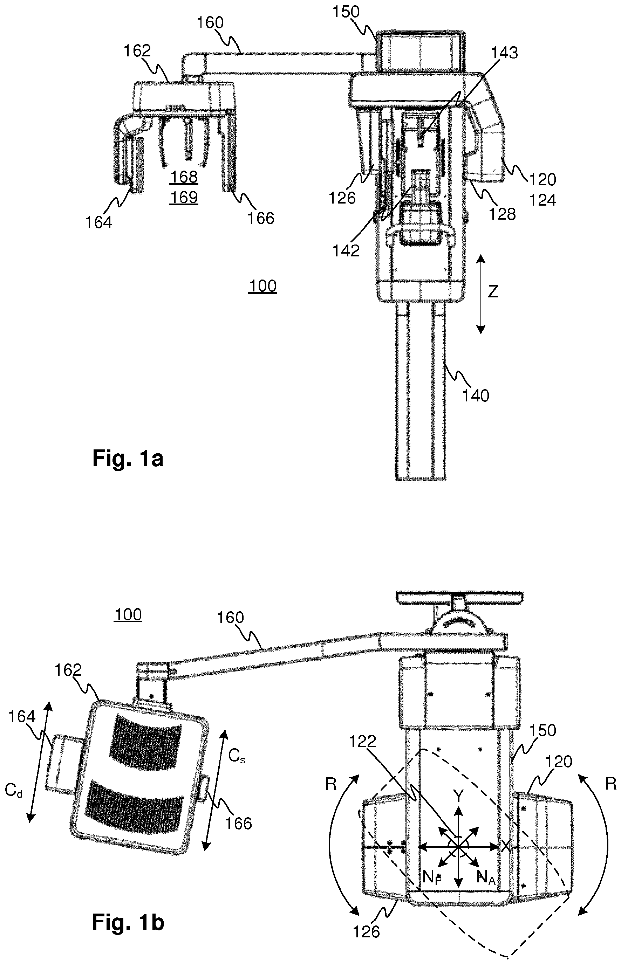

Figures 1a-1b represent a modern digital Panoramic/Cephalometric/Cone Beam Computed Tomography (CBCT) combination unit 100 having typically a column 140 that includes an up and down movement Z to adapt a height of the unit 100 to a height of a patient for Panoramic, Cephalometric, and CBCT imaging modes.

An upper shelf 150 of the unit 100 is attached to the column 140 e.g. with a fixed joint. The upper shelf 150 supports a rotating part 120.

The rotating part 120 - so-called gantry - has typically a form of a letter C incorporating an X-ray source 124 on one end and an X-ray imaging detector unit 126 on the other end. The rotating part 120 rotates R typically up to 400 degrees around a rotation axis 122.

The X-ray source 124 is common for all the three imaging modes and an X-ray beam limiting device 128 is attached in front of the X-ray source 124.

The detector unit 126 can consist of one or two detectors. The one-detector unit 126 can comprise one Cephalometric detector, which enables also the Panoramic imaging, one Panoramic/CBCT/Cephalometric combination detector, or one one-shot detector configured to be used in Cephalometric imaging. In two-detector cases, the detector unit 126 can comprise one Cephalometric detector, which enables also the Panoramic imaging, and the CBCT detector. There are several ways to attach the detectors in respect to each other and to change the detector that is located within an X-ray beam.

During imaging the beam limiting device 128 controls a size and shape of the X-ray beam so that it matches needs of a selected imaging mode, selected image size, and related detector size.

The rotation axis 122 fixes the rotation part 120 to the upper shelf 150 and it is typically attached to at least one linear movement so that the rotation axis 122 and, thus, a rotation center of the rotation part 120 in respect to the upper shelf 150 can be adjusted along a Y-movement that is parallel to the upper shelf 150 during the imaging. Furthermore, there can be a second linear X-movement perpendicular to the first one so that the rotation axis 122 can be positioned within a plane defined the linear movements X, Y.

In addition, there can be even a third N-movement that moves a fixing point of the rotation axis 122 in respect to the rotation part 120. Moving the rotation axis 122 along the X-ray beam N_{A} may be used to change a magnification within the Panoramic and CBCT imaging modes. Moving the rotation axis 122 perpendicular to the X-ray beam N_{P} enables a change between offset scanning and symmetrical scanning in the CBCT imaging, thus, affecting the Field Of View (FOV).

A Cephalometric arm 160 is used to attach a Cephalometric head 162 to the unit 100. It has typically a dedicated X-ray imaging detector 164 at one end and a secondary collimator 166 at the other end. Between these two main parts 164, 166 hang a Cephalometric patient positioning support parts 168, 169, which consist of ear rods 168 and a nose (nasion) support 169. Patient's head is supported from an outer part of an ear canal with the ear rods 168 and from a nose using the corresponding support 169.

The Cephalometric X-ray detector 164 is attached to the head 162 with a C_{d}-movement that moves the detector 164 perpendicularly to the X-ray beam. Alternatively, it is possible to perform the Cephalometric imaging by a one shot technique, when the detector 164 is sufficiently large.

The Cephalometric secondary collimator 166 is also attached to the head 162 with a Cₛ-movement that is parallel to the C_{d}-movement and, thus, also perpendicular to the X-ray beam.

The support parts 168, 169 are attached to the head 162 in a manner that enables them to rotate to two main imaging positions: the lateral and posterior anterior (PA) projections. The lateral projection is basically a side view and the PA projection is from a back-to-front view of a scull.

For Panoramic and CBCT imaging, a patient is typically supported by means of a lower shelf 142 and possibly also by means of a temple support 143. The support points are typically a tip of a chin and a forehead or temple of a patient.

The Panoramic imaging the unit 100 uses the rotation R and linear X-, Y-, or both X- and Y-movements during the scan resulting a Panoramic image. Furthermore, depending on the used sensor technology, the image is clocked out using Time Delay Integration (TDI) or full frame read-out mode of the detector. The Panoramic (sharp) layer is defined by the velocities of the movements and, in the case of TDI, the readout rate of the Panoramic detector. When using a full frame detector, the final shape of the layer is calculated on the computer after the scan. Rotation angle is typically about 270 degrees.

In the unit 100 the CBCT imaging is typically implemented by using a rotation movement R and reading out the CBCT detector with a full frame mode. Thus, projection X-ray images of the Region Of Interest (ROI) are typically produced in a way that the center of the ROI and the rotation movement R coincide. The effective rotation angle (aperture) is ranging typically from approximately 180 to 360 degrees depending on the unit 100.

In the Cephalometric imaging the patient is supported to a patient positioning structures 168, 169 located at the Cephalometric head 162 of the unit 100. The X-ray beam is arranged to scan the patient's head with a combination of rotation R and linear Y-movement. The beam is then further collimated by the secondary collimator 166 and finally captured by the Cephalometric detector 164, which both move in synchronism with the beam.

### Summary

One object of the invention is to eliminate drawbacks of the known Panoramic/Cephalometric/Computed Tomography (CT) combination units and to provide a cheaper and more compact X-ray imaging unit for medical imaging.

One object of the invention is fulfilled by providing an X-ray imaging unit, a method, a computer program, and a tangible non-volatile computer readable medium according to the independent claims.

One embodiment is an X-ray imaging unit for medical imaging. The unit comprises a column, a rotating part comprising a first X-ray source and an X-ray imaging detector unit, and an upper shelf for supporting the rotating part. The first X-ray source and the detector unit are configured to provide an image by means of at least a rotational movement around a rotation axis of the rotating part. The upper shelf enables the rotating part to move with respect to the upper shelf by means of a linear movement. The upper shelf is also attached to the column with a pivoting joint. The pivoting joint enables a pivot movement of the upper shelf around the column. The rotating part also comprises a secondary collimator that provides a cephalometric image together with a second X-ray source of the unit and the detector unit.

The term "medical imaging" refers to e.g. a dental, extraoral, oral, maxillofacial, or ears, nose, and throat imaging.

One embodiment is a method for medical imaging by means of the X-ray imaging unit, which is in accordance with the previous unit embodiment. The method comprises a step of using a secondary collimator of a rotating part of the unit to provide a cephalometric image together with a second X-ray source and an X-ray imaging detector unit of the unit.

One embodiment is a computer program configured to perform the method, which is in accordance with the previous method embodiment, when it is run in a computer. The program comprises a code for using a secondary collimator of a rotating part of the unit to provide a cephalometric image together with a second X-ray source and an X-ray imaging detector unit of the unit.

One embodiment is a tangible non-volatile computer readable medium that comprises the computer program, which is in accordance with the previous program embodiment.

Further embodiments are defined in the dependent claims.

The features recited in depending claims are mutually freely combinable unless otherwise explicitly stated.

The definitions of the below-defined verbs and terms shall be applied, unless a different definition is given in the claims or elsewhere in this description/specification.

The verb "to comprise" is used in this document as an open limitation that neither excludes nor requires the existence of unrecited features. The verbs "to in-clude" and "to have/has" are defined as to comprise.

The terms "a", "an" and "at least one", as used herein, are defined as one or more than one and the term "plurality" is defined as two or more than two. The term "another", as used herein, is defined as at least a second or more.

The term "or" is generally employed in its sense comprising "and/or" unless the content clearly dictates otherwise.

### Brief description of the figures

The embodiments of the invention will be described with reference to the accompanying figures, in which
- figures 1a-1b: represent a known digital Panoramic/Cephalometric/CT combination unit from the front and from above,
- figure 2a: represents an X-ray imaging unit for a medical imaging and its main parts and movements,
- figures 2b-2c: represent an X-ray imaging unit and a patient in Panoramic/CT and Cephalometric imaging positions and during imagings,
- figure 2d: represent a Cephalometric collimator used in the Cephalometric imaging, and
- figure 2e: represent functional elements of the X-ray imaging unit.

### Detailed description of the figures

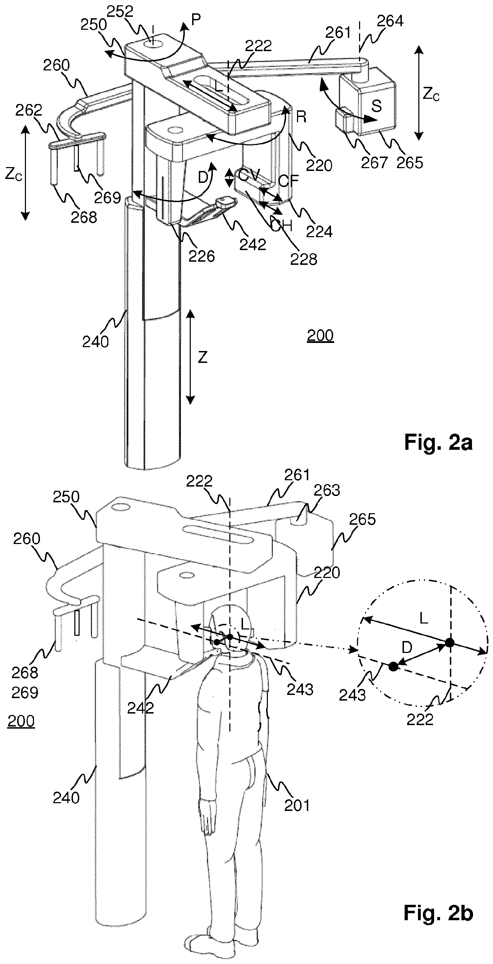

Figure 2a represents main parts of an X-ray imaging unit 200, which can be used in a medical imaging, e.g. in an extraoral dental imaging.

The unit 200 comprising a rotating part (gantry) 220, which comprises an X-ray unit comprising a first X-ray source 224. An X-ray imaging detector unit (head) 226 is attached to the rotating part 220. A position of the detector unit 226 can be adjustable, e.g. it is rotable or movable in a linear fashion. The X-ray source 224 and/or the detector unit 226 provides e.g. a Panoramic, CT, or Cephalometric image by means of at least a rotational movement R around a rotation axis 222 of the rotating part 220. The R-movement of the rotating part 220 is e.g. up to 400 degrees around the rotation axis 222.

The rotating part 220 comprising a rotating motor, which is configured to rotate the rotating part 220 by means of rotation means (not shown). Alternatively, the rotating motor can be in an upper shelf 250 of the unit 200.

The rotating part 220 has e.g. a form of a letter C and the X-ray source 224 is on one end of the rotating part 220. The X-ray source 224 is common for two imaging modes - the Panoramic imaging and CT imaging, e.g. the CBCT imaging, where an X-ray beam is a cone-shaped beam. In alternative CT imaging techniques, the X-ray beam can be a pyramidal-shaped beam, half-moon -shaped cone beam, or other shaped beam.

In addition, the X-ray source unit comprising a beam limiting device 228 for the X-ray source 224 and a beam limiting motor configured to adjust the device 228. During imaging the device 228 controls the size and shape of the X-ray beam so that it matches the needs of a selected imaging protocol, selected image size, and related detector size.

On the other end of the rotating part 220 is the detector unit 226, which can comprise e.g. one or two X-ray detectors (not shown). The one-detector unit 226 can comprise one Panoramic detector, one Cephalometric detector, which enables also the Panoramic imaging, one Panoramic/CT combination detector, one Panoramic/CT/Cephalometric combination detector, or one detector configured to be used in Panoramic/CT imaging and in one-shot Cephalometric imaging. The one-detector unit 226 can be adjustable, e.g. by rotating it relative to the rotating part 220 so that the detector of the one-detector unit 226 can be positioned preferably perpendicularly to the used X-ray source 224, 265 and/or by moving it in a linear fashion relative to the rotating part 220 for adjusting a distance between the detector and the X-ray source 224 in Panoramic/CT imaging.

In two-detector cases, the detector unit 226 can comprise one Panoramic detector and one CT detector, or one Cephalometric detector, which enables also the Panoramic imaging, and the CT detector. In the two-detectors unit 226 the detectors are arranged e.g. successively in Panoramic imaging, whereupon the Panoramic or Cephalometric detector is arranged as a front detector for arranging magnification ratio for the imaging mode, and the CT detector as a rear detector. The swap of the detectors is arranged so that the front detector moves aside by means of moving means, e.g. a rail and a rotator configured to move along the rail and to rotate so that the front detector slides e.g. next to a rear detector, when it is necessary to use the rear detector in CT imaging or the front detector in Cephalometric imaging. Alternatively, the front detector can be moved to another position relative to the rear detector in Cephalometric imaging. The place of the front detector in Cephalometric imaging dependes on how it is displaced by means of the swap movement, and the R- and L-movements relative to the used X-ray source 265. The Cephalometric detector can be positioned preferably perpendicularly to the used X-ray source 265. The front detector return similarly by sliding, when it is necessary to move it back to the front position.

The rotating part 220 can comprise a detector motor configured to move at least one detector by means of the moving means, if the detector unit 226 comprising separate detectors for the Panoramic and CT imaging.

In addition, the unit 200 comprising a column 240 for adapting a height Z of the unit 200 - and the rotating part 220. The column 240 comprises height adapting means (not shown) comprising e.g. a height motor, and telescopic or counterweighted means configured to be driven by the height motor, for providing an up/down movement Z to adapt the height of the rotating part 220 to the height of the patient 201 for the Panoramic, Cephalometric, or CT imaging modes. The height adapting means can realize the Z-movement e.g. as a telescopic or counterweighted movement.

A lower shelf 242 is attached to the column 240 and the rotating part 220 is configured to be positioned over the lower shelf 242 during the Panoramic and CT imaging. The lower shelf 242 is used for positioning a patient 201 for the Panoramic and/or CT imaging and for supporting the patient 201 e.g. from a tip of the patient's 201 chin during the imaging.

Alternatively, when the unit 200 comprising a seated patient's 201 positioning system (not shown), the Z-movement is realized e.g. by adapting in the Z-direction the height of at least one of the followings: a chair, the lower shelf 242, and the column 240.

The lower shelf 242 can also comprise a head support (not shown), which supports e.g. the patient's 201 forehead and/or temple in the Panoramic/CT imaging position.

The unit 200 comprising said upper shelf 250, which supports the rotating part 220. The upper shelf 250 is attached to e.g. an upper end of the column 240 with a pivoting joint (means) 252, which enables a pivot movement P of the upper shelf 250 around the column 240 and in respect to a lower shelf 242 so that the rotating part 220 is over e.g. the lower shelf 242.

The upper shelf 250 comprising pivot movement means (not shown), which comprising e.g. a pivot motor configured to pivot the upper shelf 250 around the column 240 by means of the pivoting joint 252.

The upper shelf 250 comprising linear movement means (not shown), e.g. a linear conveyor configured to support the rotation means of the rotating part 220 and to enable the rotating part 220 to rotate around the rotation axis 222, at least one rail and/or track configured to guide the linear conveyor in the upper shelf 250, and a linear motor configured to drive the linear conveyor along the at least one rail and the upper shelf 250, which enable the rotating part 220 and the rotation means to move with respect to the upper shelf 250 by means of a linear movement L. The linear movement means of the upper shelf 250 can be provided so that L-movement in a plane of the upper shelf 250 is a direct linear movement, e.g. it is parallel to the upper shelf 250 or it is in a certain angle with respect to the parallel direction, or the L-movement in the plane of the upper shelf 250 is a non-direct linear movement having e.g. a curved path or a devious path.

The rotation means attach the rotating part 220 to the upper shelf 250.The rotation means are able to move at least one L-movement so that the axis 222 and, thus, the rotation center in respect to the upper shelf 250 can be adjusted along the L-movement. Thus, the axis 222 can be positioned within a plane defined by the P-movement of the upper shelf 250 and the L-movement of the rotating part 220 during the imaging.

The L- and P- movements of the rotating part 220 during the imaging increase a number of imaging modes and means in the unit 200, e.g. a use of a virtual rotation axis (center) of the rotating part 220 in e.g. Panoramic and CT imaging, when there is available an increased number of movements in the unit 200 during a radiation.

The virtual rotation axis enables e.g. a use of constant magnification in the Panoramic imaging mode.

The mechanical rotation axis (center) 222 is deviated from a center line 243 of the lower shelf 242 - and the patient 201 - with the P-movement. A deviation D, which is represented in figure 2b, is maximal at a beginning of the scanning (radiating) process. When the rotating unit 220 starts to rotate by means of the R-, L-, and P-movements around the virtual rotation axis, the rotation axis 222 is shifted to the center line 243, whereupon the deviation D is minimal, i.e. non-existent, at a middle of the scanning. Then, the deviation D starts to increase in an opposite direction, when the rotating unit 220 continues the scanning, and it obtains its maximum again at an end of the scanning. This shift in the deviation D enables the magnification ratio remain the same, i.e. constant, throughout the scanning.

In addition, the virtual rotation axis enables a flexible selection between the symmetrical and offset scanning geometries in the CT imaging mode.

In the symmetrical scanning process, the virtual rotation axis is positioned on a center line of the X-ray beam. During the scanning, the rotation axis 222 and the rotating unit 220 move by means of the R-, L-, and P-movements e.g. approx.180 degrees around the virtual rotation axis, which is in a middle of the FOV.

In the offset scanning process, which provides a larger FOV than in the symmetrical scanning process, the virtual roation axis is positioned on one edge of the X-ray beam. During the scanning, the rotation axis 222 and the rotating unit 220 move by means of the R-, L-, and P-movements e.g. approx. 180 degrees around the virtual rotation axis. Then, the virtual rotation axis is positioned on another edge of the X-ray beam, and rotation axis 222 and the rotating unit 220 move by means of the R-, L-, and P-movements e.g. approx. 180 degrees around the virtual rotation axis and the FOV in opposite direction in order to gather sufficient amount of imaging data. Alternatively, the virtual rotation axis may remain at the same position during the scanning, and the rotation axis 222 and the rotating unit 220 move by means of the R-, L-, and P-movements e.g. approx. 360 degrees around the virtual rotation axis in one direction.

In addition, it is possible to design much lighter and thinner upper shelf 250, whereupon the unit 200 provides a smaller footprint when using the P-movement instead of using the linear X-movement, which widens the upper shelf 250, and the Np-movement, which widens the rotation part 220.

The X-ray source 224 on the one end of the rotating part 220 typically weights more than the detector unit 226 on the other end. As a result, a movement of the center of the gravity of the rotating part 220 can cause a varying load to a joint construction (not shown) of the rotating part 220, which comprising the linear movement means, so that the rotating part 220 wobbles during the imaging and, thus, reduces the image quality.

In order to eliminate these problems, the upper shelf 250 comprises a controlling arrangement (not shown) that enables the R-movement of the rotating part 220 in relation to the upper shelf 250 so that the axis 222 travels substantially through the center of the gravity of the rotating part 220, which, in turn, stays in a neutral axis of the joint construction of the rotating part 220 during the imaging. A virtual rotation axis of the rotating part 220 is achieved by synchronizing R-, L-, and P-movements during the scanning.

The controlling arrangement removes any torque applied to the joint construction and increases an image quality by removing artifacts caused by wobbling.

In addition, the controlling arrangement enables to produce a lighter, cheaper, and slender structure of the rotating part 220 and its joint construction.

In addition, the unit 200 comprising on one side of the column 240 a first Cephalometric arm 260 that has a certain first length. The arm 260 attaches a Cephalometric head (unit) 262 to the unit 200 at a certain first distance that corresponds with the first length from the column 240.

The Cephalometric head (support) 262, which has a significantly simpler structure than in traditional Cephalometric units, comprises a Cephalometric patient support means 268, 269, e.g. two ear rods 268 and a nose (nasion) support 269, for supporting the patient 201 to be imaged. The patient's 201 head is supported e.g. from an outer part of a ear canal with the ear rods 268 and from the nose using the support 269. The ear rods 268 and nose support 269 can be attached to the Cephalometric head 262 in a manner that enables them to rotate e.g. to two main imaging positions: lateral and PA projections. The lateral projection is basically a side view and the PA projection is from back to front view of a scull of the patient 201.

The ear rods 268 can be tiltable or rotable ear rods having a down position, where the ear rods 268 support the patient 201, and an up position, where it is possible to place the patient 201 in the Cephalometric imaging position or where the patient 201 can depart from the the Cephalometric imaging position, when the tilted or rotated ear rods 268 in the up position provide a clear passage of the patient 201.

In addition, the unit 200 comprising on other side of the column 240 a second Cephalometric arm 261 that has a certain second length. The arm 261 attaches a second X-ray source 265, which is used in the Cephalometric imaging, to the unit 200 at a certain second distance that corresponds with the second length from the column 240. The X-ray source 265 comprising a beam limiting device 267 for the Cephalometric imaging and it is attached to the X-ray source 265. The X-ray source 265 can be configured to rotate around a rotation axis 264 by means of rotation means 263 configured to perform a scanning movement S. The axis 264 of the X-ray source 265 is in line with a focal spot of the X-ray source 265 so that it passes through the focal spot. The arm 261 or the X-ray source 265 comprising a rotating motor, which is configured to rotate the X-ray source 265 around the axis 264, which coincides with the focal spot of the X-ray source 265.

The arms 260, 261 can be separate arms attached to the column 240 or, alternatively, it is possible to use one arm 260, 261, which comprising the Cephalometric head 262 in its one end and the X-ray source 265 with the beam limiting device 267 in the other end of the single arm 260, 261.

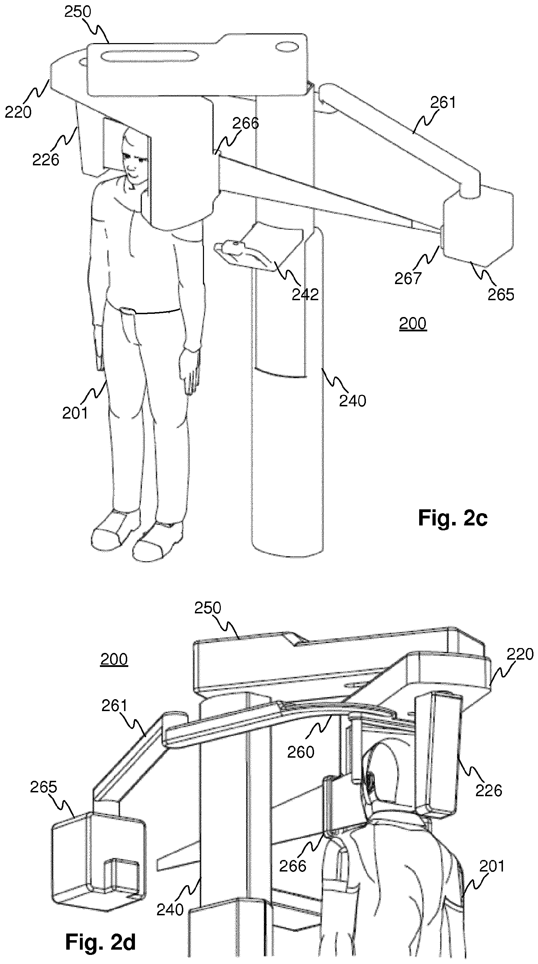

Figure 2d represents that how the Cephalometric, combination, or one-shot detector of the detector unit 226 attached to the rotating part 220 is used also with the Cephalometric imaging.

In addition, figure 2d represents that the rotating part 220 can comprise a Cephalometric (secondary) collimator 266, which is used in the Cephalometric imaging. The Cephalometric collimator 266 is attached e.g. to a side of the rotating part 220.

In addition, the rotating part 220 can comprise a detector motor configured to rotate the detector unit 226 for the Cephalometric imaging and a collimator motor configured to adjust a position (height) of the Cephalometric collimator 266 in the Z-direction. Alternatively, it is possible that the beam limiting motor or the collimator motor is configured to adjust both the device 228 and the Cephalometric colllimator 266.

The rotating part 220 is driven over the Cephalometric head 262, e.g. with the P-, R-, and L-movements, so that the detector unit 226 and the Cephalometric collimator 266 are positioned for the Cephalometric imaging.

The X-ray source 265 can be configured to provide, together with e.g. the detector unit 226 and the Cephalometric collimator 266 in the rotating part 220, a Cephalometric image from the positioned patient 201, when it is rotated around the axis 264 by means of the S-movement, and the detector unit 226 and the Cephalometric collimator 266 are arranged to move e.g. by means of at least one of the P-, R-, and L-movements of the rotating part 220. Alternatively, the scanning movement of the x-ray beam - e.g. a linear S-movement - can be performed by moving the beam limiting device 267 of the X-ray source 265.

If the one-shot detector is used, the detector unit 226 and the Cephalometric collimator 266 are positioned by means of at least one of the P-, R-, and L-movements, but the image can be taken without these movements and the S-movement.

Thus, there is no need for a dedicated holder or the Cₛ-movement for the detector unit 164 and the C_{d}-movement of the Cephalometric collimator 166, when the scanning movement is executed with e.g. the P-, R-, L-, and S-movements.

The arms 260, 261 can be arranged so that a height of the Cephalometric head 262 with the ear rods 268 and nose support 269, relative to the X-ray source 265, is fixed.

However, the fixed height may cause problems, because an anatomy of patients 201 varies - the vertical distance where ear openings are located compared to patient's 201 shoulders differs significantly from one patient 201 to another. Thus, either the patient 201 is located too low in the resultant Cephalometric image showing only upper vertebras or the patient 201 is located so high in the images that the shoulder of the patient 201 touches the detector unit 226, which is a problem especially with a scanning. Furthermore, the preferred Cephalometric imaging geometry requires that the focal spot and the tips of the ear rods 268 are at the same (horizontal) axis.

In order to eliminate these problems, variable length ear rods 268 can be used while keeping the arms 260, 261 fixed height relative to each other.

Alternatively or in addition, in order to eliminate these problems, the unit 200 can comprise Cephalometric height adjusting means (not shown) that are configured to independently adjust the height - in respect to the column 240 - of the arms 260, 261 that support the Cephalometric head 262 at the one end and the X-ray source 265 on the other end.

When the operator has adjusted the height of the arms 260, 261 by means of an up/down Zc-movement, the focal spot follows the tips of the ear rods 268 automatically and, thus, the geometry (ear rod tip to focal spot line) remains intact. Yet, the detector unit 226 and the Cephalometric collimator 266 on each side of the patient 201 take their height from the column 240 and, thus, are on a different height in respect to the ear rods 268 and the patient 201 than before the adjustment.

The Cephalometric height adjusting means provides a way to adapt an exposed area to a given anatomy of the patient 201 by enabling an operator (user) to adjust the height of the patient 201 without compromising the geometry.

The movements of unit 200 are simple, because the traditional X-movement, and the C_{d}- and Cₛ-movements of the detector unit 226 and the secondary collimator 266 in the Cephalometric head 262 are replaced by using the P-movement instead. The movements are carried out using the L-movement and the P-movement of the upper shelf 250.

In addition, the structure of unit 200 is simplier and cheaper, when using the P-movement, because the Cephalometric imaging can optionally be implemented by using only one "non-detachable" detector unit 226 so that it is avoided a breaking of the detector unit 226 when there is no need to remove it from a holder of the rotating part 220 to detach it to a holder of the Cephalometric head 262 when changing the imaging mode from the Panoramic/CT mode to the Cephalometric mode. The detector for the Panoramic imaging in the detector unit 226 can be rotated from the Panoramic imaging position to the Cephalometric imaging position so that it is possible to use the same detector in both Panoramic and Cephalometric imaging.

In addition, the structure of unit 200 provides a simple workflow when e.g. the change from the Panoramic/CT mode to the Cephalometric mode - the movement of the rotating part 220 from the Panoramic/CT imaging position to the Cephalometric position without changing the detector unit 226 from one holder to other holder - is automatized, whereupon a manual work during the work flow and time needed for the work flow decrease.

It is also possible that the unit 200 comprising the upper shelf 250 that pivots around the column 240 and the rotating part 220 that is configured to be positioned by means of the above-described L- and P-movements for providing the Panoramic and/or CT imaging, but the Cephalometric head 262 is similar to the known Cephalometric head, which comprising the Cephalometric detector, the secondary collimator, and the patient positioning support parts.

The Cephalometric imaging is provided by means of the X-ray source 224 of the rotating part 220, and the secondary collimator and the Cephalometric detector of the Cephalometric head 262. The X-ray source 224 is arranged to scan the patient's 201 head with the R- and L-movements. The X-ray beam is collimated by the secondary collimator and captured by the Cephalometric detector, which are synchronized with the X-ray beam.

Figure 2b represents a positioning of the patient 201 during the Panoramic/CT imaging.

The patient 201 is supported by the lower shelf 242 and possibly to the head support of the unit 200 in a Panoramic/CT imaging position, where the rotating part 220 is over the lower shelf 242.

If the upper shelf 250 as well as the rotating part 220 are in a different position than the Panoramic/CT imaging position - in a Cephalometric imaging position or in an intermediate position between e.g. the Panoramic/CT and Cephalometric imaging positions - the upper shelf 250 is moved from that position to the Panoramic/CT imaging position by the P-movement and, then, the rotating part 220 is further adjusted by the R- and L-movements so that the rotating part 220 is ready for the Panoramic/CT imaging.

In addition, the rotating part 220 can have a patient postioning position, where the X-ray source 224 or the detector unit 226 do not prevent the positioning of the patien 201 to the Panoramic/CT and/or Cephalometric imaging positions when the rotating part 220 is over the lower shelf 242 or the Cephalometric head 262. The patient postioning position can be accomplished by the R-movement so that the rotating part 220 is rotated such position, where it is possible to place the patient 201 to the Panoramic/CT and/or Cephalometric imaging positions or to remove the patient 201 by moving the patient's 201 head between the X-ray source 224 and the detector unit 226. Alternatively, it is possible to realize the patient postioning position by means of the P-movement, whereupon the whole rotating part 220 is moved away from the Panoramic/CT and/or Cephalometric imaging positions, when the patient 201 is positioned.

The positioned X-ray source 224 and the detector unit 226 are configured to provide a Panoramic image when the rotation axis 222- a rotation center of the rotating part 220 - is positioned by at least one of the P - and L- movements.

Depending on the sensor technology used, the image can be clocked out using TDI or full frame read-out mode of the detector. In the TDI mode, the image is read out one column at a time, whereas in the full frame mode, the image is read out whole image frame at a time. The Panoramic (sharp) layer is defined by the velocities of the movements and, in the case of TDI, the readout rate of the Panoramic detector. When using a full frame detector, the final shape of the layer is calculated on the computer after the scan. Rotation angle is typically about 270 degrees.

During the CT imaging, the patient 201 is also supported to the lower shelf 242 and possibly to the head support of the unit 200 in the Panoramic/CT imaging position. The X-ray source 224 and the detector unit 226 are configured to provide a CT image when the detector unit 226 is attached to the rotating unit and the rotation center of the rotating part 220 is positioned so that it can coincides the ROI.

The positioned X-ray source 224 and the detector unit 226 are configured to provide a CT image, e.g. CBCT image, when the detector unit 226 is attached to the rotating unit 220, and the rotation axis 222 is positioned by at least one of the R-, L-, and P-movements during the CT imaging.

When it is used a symmetric imaging geometry, the CT imaging can be carried out by using only the R-movement and reading out the CT detector with a full frame mode. Alternatively, CT imaging can be carried out by using the P-, R-, and L-movements, when it is used the controlling arrangement in the upper shelf 250, for positioning the virtual rotation axis of the rotating part 220 so that it coincides the ROI. Thus, projection X-ray images of the ROI are produced in a way that the center of the ROI and the R- movement coincide. The effective rotation angle (aperture) is ranging e.g. from approximately 180 to 360 degrees depending on the unit 200.

When it is used an offset imaging, the CT imaging can be carried out by scanning the image by using the R-, L-, and P-movement. By driving these R-, L-, and P-movements in synchronism, the effective center of the rotation can be deflected to the side of the beam and, thus creating an offset geometry.

The offset scanning can be provided by a first "solid" offset geometry and a full 360 degree rotation of the CT detector.

Alternatively, the offset scanning can be provided by a second offset geometry, where the patient 201 is imaged by scanning an essentially maximal first imaging offset by approximately 180 degree rotation of the detector in a first imaging direction. Then, the detector is displaced to the other side of the rotation center to obtain an essentially maximal second imaging offset by approximately 180 degree rotation of the detector in a second imaging direction, which is opposite to the first direction. Alternatively, the detector is rotated to the starting position, displaced to the other side of the rotation center, and, then, scanning the the essentially maximal second imaging offset by approximately 180 degree rotation in the first direction.

Alternatively, the offset scanning can be provided by a third offset geometry, where the patient 201 is imaged by a first imaging offset, where the edge of the X-ray beam area touches the rotation center, and by 360 degree rotation of the detector. Next, the detector and the X-ray source 224 are displaced parallel in such a way that the X-ray beam area moves away from the rotation center so it hits or slightly overlaps the previously imaged area. Then, the detector is rotated 360 degree for completing a second imaging offset.

The unit 200 provides same versatility in the CT imaging geometry by means of the R-, L-, and P-movements instead of the R-, L, X-, and N-movements required in imaging and patient postioning by some conventional systems.

Figure 2c represents a positioning of the patient 201 during the Cephalometric imaging.

In the Cephalometric imaging position, where the rotating part 220 is over the patient support means 268, 269 located at the Cephalometric head 262, the patient 201 is supported to the patient support means 268, 269.

If the upper shelf 250 as well as the rotating part 220 are in a different position than the Cephalometric imaging position - e.g. in a Panoramic/CT imaging position or in an intermediate position between the Panoramic/CT and Cephalometric imaging positions - the upper shelf 250 is moved from that position to the Cephalometric imaging position by the P-movement, and then the rotating part 220 is further adjusted by the R- and L-movements so that the rotating part 220 is ready for the Cephalometric imaging.

The positioned X-ray source 265 is configured to scan the supported patient 201 by means of the beam limiting device 267 attached to the X-ray source 265 and by means of the S-movement. The detector unit 226 - and the rotating part 220 - is configured to move synchronously with the X-ray source 265 by the R-, L-, and P-movements during the Cephalometric imaging.

The X-ray beam from the X-ray source 265 is arranged to scan the patient's 201 head by rotating the X-ray source 265 and the beam limiting device 267 with the S-movement around the axis 264. Alternatively, the S-movement can be performed by moving e.g. linearly the beam limiting device 267. It is also possible that the S-movement is provided as a vertical scanning movement instead of the horizontal S-movement, if the detector of the detector unit 226 used in Cephalometric imaging is positioned horizontally. Alternatively, Cephalometric imaging can be performed without the S-movement if a sufficiently large detector (so-called one-shot detector) is used for the one-shot Cephalometric image.

The beam is then further collimated by the Cephalometric collimator 266 and finally captured by the synchronously moved Cephalometric or combination detector in the detector unit 226.

The unit 200 simplifies the movements during the Cephalometric imaging, because no additional movement means are needed for the Cephalometric collimator 266 and the detector of the detector unit 226.

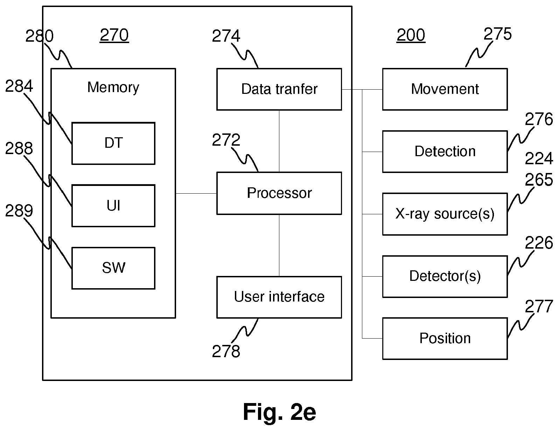

Figure 2e represents the functional elements of the unit 200.

The unit 200 comprises a control unit (control panel) 270 that is configured to control the unit 200, and its above-described movements and imaging processes. The control unit 270 are attached e.g. to the column 240.

The control unit 270 comprises at least one processor (portion) 272 for performing user and/or software initiated instructions and for processing data, and at least one memory (portion) 280 for storing and maintaining data, e.g. instructions, softwares, and data files.

In addition, the control unit 270 comprises a data transfer portion 274 for sending control commands to e.g. the pivot, linear, height, rotating, detector, beam limiting, and collimator motors, drivers, or other means (motors, devices) 275 configured to provide the movements of the parts of the unit 200, and/or receiving data from measuring devices or other detection devices 276 configured to detect the function of parts of the unit 200.

In addition, the data transfer portion 274 is also configured to send control commands to the at least one of followings: at least one of X-ray source 224, 265, the detector unit 266, and positioning means 277, e.g. at least one laser, camera, or other indication means, configured to facilitate a positioning of the patient 201 in the Panoramic imaging position and/or CT imaging position by incating a correct positioning of the patient 201. The data transfer portion 274 is also configured to receive information from at least one of the followings: the at least one X-ray source 224, 265, the detector unit 226, and the positioning means 277.

In addition, the control unit 270 comprises a user interface portion 278 comprising e.g. at least one of the followings: at least one function key, a touchscreen, and a wired or wireless remote controller, for inputting control commands, and for receiving information and/or instructions.

The at least one memory 280 comprises at least a data transfer application 284 for controlling the data transfer portion, a user interface application 288 for controlling the user interface portion, and a computer program (code) 289 for controlling the function of the unit 200, e.g. at least the movement devices 275, detection devices 286, the at least one X-ray source 224, 265, the detector unit 226, and positioning means 277. In addition, the computer program 289 can control e.g. imaging parameters, imaging sizes, and imaging modes.

The at least one memory 280 and the computer program 289 are configured to, with the at least one processor 272, cause the unit 200 at least to provide actions described in context of figures 2a-2d

The computer program 289 can be a computer program product that comprises a tangible, non-volatile (non-statutory) computer-readable medium bearing a computer program code 289 embodied therein for use with a computer (control unit 270).

## Claims

1. An X-ray imaging unit (200) for medical imaging, comprising a column (240),
a rotating part (220) comprising a first X-ray source (224) and an X-ray imaging detector unit (226),
an upper shelf (250) for supporting the rotating part,
a first arm (260) with an attached cephalometric head (262) having a cephalometric patient support (268, 269) for supporting a patient (201) to be imaged,
a second arm (261) with an attached a second X-ray source (265), and a control unit (270) configured to control the X-ray imaging unit,
wherein the first X-ray source and the detector unit are configured to provide an image by means of at least a rotational movement (R) around a rotation axis (222) of the rotating part,
wherein the upper shelf is configured to enable the rotating part to move with respect to the upper shelf by means of a linear movement (L) and the upper shelf is attached to the column with a pivoting joint (252),
wherein the pivoting joint enables a pivot movement (P) of the upper shelf around the column,
wherein the rotating part is configured to be positioned by the linear and pivot movements during the imaging, and
wherein the rotating part further comprises a cephalometric collimator (266) and is configured to be positioned over the cephalometric head by the pivot movement so that the second X-ray source and the detector unit are configured to provide a cephalometric image.

2. The unit according to the preceding claim, wherein the cephalometric collimator is attached to a side of the rotating part.

3. The unit according to any of the preceding claims, wherein the rotating part comprises a detector motor that rotates the detector unit and a collimator motor that adjusts a height of the cephalometric collimator.

4. The unit according to any of the preceding claims, wherein the second X-ray source comprises a beam limiting device (267) and wherein the second X-ray source is configured to perform a scanning movement (S) by rotating around a rotation axis (264) or by moving the beam limiting device.

5. The unit according to any of claims, wherein the second X-ray source is configured to perform a scanning movement (S) by moving a beam limiting device (267) attached to the second X-ray source, and wherein the detector unit is moved synchronously with the second X-ray source by the rotational, linear, and pivot movements during the production of the cephalometric image.

6. The unit according to any of preceding claims, which further comprises a lower shelf (242) for positioning the patient (201) to be imaged for a panoramic and/or computed tomography imaging, and the rotating part is configured to be positioned over the lower shelf.

7. The unit according to any of the preceding claims, wherein the first X-ray source and the detector unit together are configured to provide at least one of a panoramic image and a computed tomography image.

8. A method for medical imaging by means of the X-ray imaging unit (200) according to any of the preceding claims, comprising steps of
positioning the rotating part (220) over the cephalometric head (262) by the pivot movement (P) and
using the second X-ray source (265) together with the cephalometric collimator (266) and the detector unit (266) to provide the cephalometric image.

9. A computer program (289) comprising instructions, which, when executed by the control unit, cause the control unit to perform the steps of the method according to claim 8.

10. A tangible, non-volatile computer-readable medium comprising the computer program (289) according to claim 9.

## Patentansprüche

1. Röntgenbildgebungseinheit (200) für medizinische Bildgebung, umfassend:
eine Säule (240),
ein drehendes Teil (220), das eine erste Röntgenquelle (224) und eine Röntgenbild-Detektoreinheit (226) umfasst,
eine obere Ablage (250) zum Tragen des drehenden Teils, einen ersten Arm (260) mit einem daran befestigten kephalometrischen Kopf (262) mit einer kephalometrischen Patientenstütze (268, 269) zum Stützen eines abzubildenden Patienten (201),
einen zweiten Arm (261) mit einer daran befestigten zweiten Röntgenquelle (265), und
eine Steuereinheit (270), die konfiguriert ist, um die Röntgenbildgebungseinheit zu steuern,
wobei die erste Röntgenquelle und die Detektoreinheit dazu konfiguriert sind, mittels mindestens einer Drehbewegung (R) um eine Drehachse (222) des drehenden Teils ein Bild bereitzustellen,
wobei die obere Ablage konfiguriert ist, um eine Bewegung des drehenden Teils in Bezug auf die obere Ablage mittels einer Linearbewegung (L) zu ermöglichen und die obere Ablage mit einem Schwenkgelenk (252) an der Säule befestigt ist,
wobei das Schwenkgelenk eine Schwenkbewegung (P) der oberen Ablage um die Säule ermöglicht,
wobei das drehende Teil so konfiguriert ist, dass es durch die Linear- und Schwenkbewegungen während der Bildgebung positioniert wird, und
wobei das drehende Teil ferner einen kephalometrischen Kollimator (266) umfasst und konfiguriert ist, um durch die Schwenkbewegung über dem kephalometrischen Kopf positioniert zu werden, so dass die zweite Röntgenquelle und die Detektoreinheit konfiguriert sind, um ein kephalometrisches Bild bereitzustellen.

2. Einheit nach dem vorhergehenden Anspruch, wobei der kephalometrische Kollimator an einer Seite des drehenden Teils angebracht ist.

3. Einheit nach einem der vorhergehenden Ansprüche, wobei das drehende Teil einen die Detektoreinheit drehenden Detektormotor und einen Kollimatormotor umfasst, der eine Höhe des kephalometrischen Kollimators einstellt.

4. Einheit nach einem der vorhergehenden Ansprüche, wobei die zweite Röntgenquelle eine Strahlbegrenzungsvorrichtung (267) umfasst, und wobei die zweite Röntgenquelle dazu konfiguriert ist, eine Scanbewegung (S) durch Drehen um eine Drehachse (264) oder durch Bewegen der Strahlbegrenzungsvorrichtung durchzuführen.

5. Einheit nach einem der Ansprüche, wobei die zweite Röntgenquelle dazu konfiguriert ist, eine Scanbewegung (S) durch Bewegen einer an der zweiten Röntgenquelle angebrachten Strahlbegrenzungsvorrichtung (267) durchzuführen, und wobei die Detektoreinheit durch die Dreh-, Linear- und Schwenkbewegungen während der Erzeugung des kephalometrischen Bildes synchron mit der zweiten Röntgenquelle bewegt wird.

6. Einheit nach einem der vorhergehenden Ansprüche, die ferner eine untere Ablage (242) zum Positionieren des abzubildenden Patienten (201) für eine Panorama- und/oder Computertomographie-Bildgebung umfasst, und das drehende Teil konfiguriert ist, um über der unteren Ablage positioniert zu werden.

7. Einheit nach einem der vorhergehenden Ansprüche, wobei die erste Röntgenquelle und die Detektoreinheit zusammen konfiguriert sind, um mindestens eines von einem Panoramabild und einem Computertomographiebild bereitzustellen.

8. Verfahren zur medizinischen Bildgebung mittels der Röntgenbildgebungseinheit (200) nach einem der vorhergehenden Ansprüche, umfassend die folgenden Schritte:
Positionieren des drehenden Teils (220) über dem kephalometrischen Kopf (262) durch die Schwenkbewegung (P) und
Verwenden der zweiten Röntgenquelle (265) zusammen mit dem kephalometrischen Kollimator (266) und der Detektoreinheit (266), um das kephalometrische Bild bereitzustellen.

9. Computerprogramm (289), das Anweisungen umfasst, die, wenn sie von der Steuereinheit ausgeführt werden, die Steuereinheit veranlassen, die Schritte des Verfahrens nach Anspruch 8 durchzuführen.

10. Materielles, nichtflüchtiges computerlesbares Medium, das das Computerprogramm (289) nach Anspruch 9 umfasst.

## Revendications

1. Unité d'imagerie à rayons X (200) pour l'imagerie médicale, comprenant
une colonne (240),
une partie rotative (220) comprenant une première source de rayons X (224) et une unité de détection d'imagerie à rayons X (226),
une étagère supérieure (250) pour supporter la partie rotative,
un premier bras (260) avec une tête céphalométrique (262) fixée ayant un support de patient céphalométrique (268, 269) pour supporter un patient (201) à imager,
un second bras (261) avec une seconde source de rayons X (265) fixée, et
une unité de commande (270) conçue pour commander l'unité d'imagerie à rayons X,
dans laquelle la première source de rayons X et l'unité de détection sont conçues pour fournir une image au moyen d'au moins un mouvement de rotation (R) autour d'un axe de rotation (222) de la partie rotative,
dans laquelle l'étagère supérieure est conçue pour permettre à la partie rotative de se déplacer par rapport à l'étagère supérieure au moyen d'un mouvement linéaire (L) et l'étagère supérieure est fixée à la colonne par une articulation pivotante (252),
dans laquelle l'articulation pivotante permet un mouvement de pivotement (P) de l'étagère supérieure autour de la colonne,
dans laquelle la partie rotative est conçue pour être positionnée par les mouvements linéaire et de pivotement pendant l'imagerie, et
dans laquelle la partie rotative comprend en outre un collimateur céphalométrique (266) et est conçue pour être positionnée sur la tête céphalométrique par le mouvement de pivotement de sorte que la seconde source de rayons X et l'unité de détection sont conçues pour fournir une image céphalométrique.

2. Unité selon la revendication précédente, dans laquelle le collimateur céphalométrique est fixé sur un côté de la partie rotative.

3. Unité selon l'une quelconque des revendications précédentes, dans laquelle la partie rotative comprend un moteur de détection qui fait tourner l'unité de détection et un moteur de collimateur qui règle une hauteur du collimateur céphalométrique.

4. Unité selon l'une quelconque des revendications précédentes, dans laquelle la seconde source de rayons X comprend un dispositif de limitation de faisceau (267) et dans laquelle la seconde source de rayons X est conçue pour effectuer un mouvement de balayage (S) en tournant autour d'un axe de rotation (264) ou en déplaçant le dispositif de limitation de faisceau.

5. Unité selon l'une quelconque des revendications, dans laquelle la seconde source de rayons X est conçue pour effectuer un mouvement de balayage (S) en déplaçant un dispositif de limitation de faisceau (267) fixé à la seconde source de rayons X, et dans laquelle l'unité de détection est déplacée de manière synchrone avec la seconde source de rayons X par les mouvements de rotation, linéaire et de pivotement lors de la réalisation de l'image céphalométrique.

6. Unité selon l'une quelconque des revendications précédentes, qui comprend en outre une étagère inférieure (242) pour positionner le patient (201) à imager pour une imagerie panoramique et/ou de tomodensitométrie, et la partie rotative est conçue pour être positionnée sur l'étagère inférieure.

7. Unité selon l'une quelconque des revendications précédentes, dans laquelle la première source de rayons X et l'unité de détection sont conçues ensemble pour fournir au moins l'une d'une image panoramique et d'une image de tomodensitométrie.

8. Procédé d'imagerie médicale au moyen de l'unité d'imagerie à rayons X (200) selon l'une quelconque des revendications précédentes, comprenant les étapes
de positionnement de la partie rotative (220) sur la tête céphalométrique (262) par le mouvement de pivotement (P) et
d'utilisation de la seconde source de rayons X (265) conjointement avec le collimateur céphalométrique (266) et l'unité de détection (266) pour fournir l'image céphalométrique.

9. Programme informatique (289) comprenant des instructions qui, lorsqu'elles sont exécutées par l'unité de commande, amènent l'unité de commande à exécuter les étapes du procédé selon la revendication 8.

10. Support tangible non volatile lisible par ordinateur comprenant le programme informatique (289) selon la revendication 9.
